# EUROPEAN PATENT SPECIFICATION

(11) **EP 0 599 282 B1**
(45) Date of publication and mention of the grant of the patent: **09.06.1999**
(21) Application number: 93118886.6
(22) Date of filing: 24.11.1993
(51) Int. Cl.: A61K 9/16, A61K 31/575

(54) **Pharmaceutical compositions containing ursodeoxycholic acid**
Pharmazeutische Zusammensetzungen enthaltend Urodeoxycholicsäure
Compositions pharmaceutiques contenant de l'acide ursodeoxycholique

(30) Priority: 27.11.1992 IT MI922721
(43) Date of publication of application: 01.06.1994
(73) Proprietor: BIOPROGRESS S.p.A., I-00165 Rome (IT)
(72) Inventor: Olivieri, Aldo, I-00165 Rome (IT); Camponeschi, Claudio, I-00136 Rome (IT)
(74) Representative: Gervasi, Gemma, Dr.

(56) References cited:
- EP-A- 0 508 312
- EP-A- 0 576 938
- WO-A-89/02738
- CHEMICAL ABSTRACTS, vol. 109, no. 10, 5 September 1988, Columbus, Ohio, US; abstract no. 79754z,
- MANUFACTURING CHEMIST AND AEROSOL NEWS vol. 41, no. 6 , 1970 pages 40 - 43 REYNOLDS A.D. 'A NEW TECHNIQUE FOR THE PRODUCTION OF SPHERICAL PARTICLES'

## Description

### Field of the invention

The present invention relates to controlled release formulations based on ursodeoxycholic acid.

### State of the art

Ursodeoxycholic acid [(3α, 5β, 7β)-3,7-dihydroxycholan-24-oic acid] is a bile acid especially suitable for the treatment of cholesterol metabolic disorders.

Controlled release formulations of ursodeoxycholic acid are already known: exemplary of systems that utilize controlled release of the active agent are compressed tablets with polymeric matrices or microcapsules coated with a polymeric film, for instance, like the compressed compositions disclosed in the document EP-A-508312. The former imply the use of cellulose derivatives which, after compression, control the release of the drug, the latter imply the use of coating films based on acrylic polymers or gelatin, which delay drug liberation. In all cases, the presence of polymers, either poorly soluble or forming slow-releasing gels, is of fundamental importance.

### Summary of the invention

It has surprisingly been found that controlled release compositions containing high amounts of ursodeoxycholic acid as active ingredient and small amounts of commonly used excipients, such as binders, plasticizers, and optionally disintegrating agents, may be prepared, with no addition of polymers that delay drug liberation, by extrusion-spheronization of the mixture of ursodeoxycholic acid and excipients to give high-density microgranules.

### Detailed description of the invention

It is an object of the present invention to provide controlled release pharmaceutical compositions for ursodeoxycholic acid oral administration, consisting of microgranules prepared by extrusion/spheronization and containing, in addition to said acid, a plasticizer, a binder and optionally a disintegrating agent.

The microgranules of the pharmaceutical compositions under the present invention are prepared by a process envisaging the steps of:
1. dry mixing ursodeoxycholic acid with appropriate amounts of plasticizer, binder and optionally disintegrating agents;
2. feeding the resulting mixture to an extruder screw;
3. simultaneously feeding demineralized water, as granulation fluid, to the extruder screw;
4. extruding the plastic mass through a die with orifices of proper diameter to obtain extruded cylinders;
5. spheronizing the extruded cylinders by feeding same to a high-speed rotating disk breaking down said cylinders to spherical microgranules having the same size as the orifices of the extrusion die;
6. drying the spheronized microgranules according to a known technique, such as for example suspending said microgranules in a fluid bed, placing same in trays to be inserted in appropriate driers, etc.

It is a further object of the present invention to provide a process for the preparation of pharmaceutical compositions in the form of high-density controlled release microgranules containing ursodeoxycholic acid, said process being based on the extrusion of a mixture consisting of the drug, a plasticizing agent, a binder, optionally a disintegrating agent and the granulation fluid, followed by spheronization of the extrudate and drying of the resulting microgranules.

To obtain microgranules of good technological properties (i.e. high density, low friability, satisfactory free flowing), the ursodeoxycholic acid is to be added with a plasticizer and a binder; the optional addition of a disintegrating agent may favour a very fast drug release.

Among the plasticizer substances providing the appropriate plasticity to the mass to be extruded, microcrystalline cellulose was found to be the most suitable for obtaining microgranules of high density and low porosity, and, therefore, with a more controllable dissolution rate.

Among binders, hydroxypropyl cellulose was found to significantly reduce microgranules friability and to hinder caking tendency during the spheronization; furthermore, said substance secures a significant modularity of the release kinetics. Other non-limitative examples of binders are hydroxypropyl methylcellulose, linear polyvinylpyrrolidone, etc.

The mixture to be extruded and spheronized may be added also with a disintegrating agent to increase the modularity of the release kinetics of the microgranules obtained by extrusion/spheronization. Non-limitative examples of disintegrating agents are cross-linked polyvinylpyrrolidone, cross-linked carboxymethylcellulose, carboxymethylamylum, etc.

Most unexpectedly, thanks to the extrusion/spheronization process, the amounts of binders and plasticizers to be added to the ursodeoxycholic acid to secure an excellent control of the technological and release properties are very low. In fact, the ursodeoxycholic acid content in the mixture to be extruded may range between 50 and 90% by wt., preferably between 70 and 90%; the plasticizer content between 5 and 50%, preferably between 10 and 30%; the binder content between 0.5 and 4%, preferably between 2 and 4%; the disintegrating agent content between 1 and 10%, preferably between 1 and 5%.

The simultaneous presence of binder, plasticizer and disintegrating agent, even in fairly low amounts in respect of the ursodeoxycholic acid, unexpectedly brings about very different release rates: an almost total ursodeoxycholic acid release from compositions containing the disintegrating agent takes few hours, while it takes at least 24 hours in case of compositions containing only binder and plasticizer. This result is secured also by the extrusion technique , which gives very compact and little porous granules, as evidenced by the density data (0.6 to 0.7 g/ml) measured on test preparations.

A further advantage of the present invention is that the microgranules prepared by extrusion-spheronization may be directly enclosed in hard gelatin capsules without addition of other excipients; consequently, a higher technological and dosing flexibility is achieved. In fact, it is enough to change the capsule size to allow the capsulation of different amounts of granules and, therefore, the preparation of differently-dosed pharmaceutical forms.

### Example 1

Ursodeoxycholic acid (604 g), microcrystalline cellulose (111 g) and hydroxypropyl cellulose (7.1 g) were mixed in a cube mixer and fed to a twin screw extruder. Demineralized water (505 ml) was added as granulation fluid and the mass, fed at a temperature of 50°C by the screw rotating at 140 rpm, was extruded through a die with 1 mm dia. orifices.

The extrudate was fed to the spheronizer drum rotating at 1000 rpm. During said operation, microcrystalline cellulose (12.3 g) was added to prevent microgranules from caking. After spheronization, microgranules were suspended in a fluid bed and dried at 70°C.

The microgranules obtained had the following by wt. per cent composition:

| | |
|---|---|
| Ursodeoxycholic acid | 83.6 |
| Microcrystalline cellulose | 15.4 |
| Hydroxypropyl cellulose | 1.0 |

### Example 2

Ursodeoxycholic acid (602 g), microcrystalline cellulose (70 g) and hydroxypropyl cellulose (5 g) were mixed and subjected to extrusion (after addition of demineralized water) and spheronization as per Example 1. The microgranules obtained after drying in a fluid bed had the following by wt. per cent composition:

| | |
|---|---|
| Ursodeoxycholic acid | 87.3 |
| Microcrystalline cellulose | 12.0 |
| Hydroxypropyl cellulose | 0.7 |

### Example 3

Ursodeoxycholic acid (604 g), microcrystalline cellulose (60 g) and hydroxypropyl cellulose (3.5 g) were mixed and subjected to extrusion (after addition of demineralized water) and spheronization as per Example 1. The microgranules obtained after drying in a fluid bed had the following by wt. per cent composition:

| | |
|---|---|
| Ursodeoxycholic acid | 88.5 |
| Microcrystalline cellulose | 11.0 |
| Hydroxypropyl cellulose | 0.5 |

### Example 4

Ursodeoxycholic acid (801 g), microcrystalline cellulose (150 g), hydroxypropyl cellulose (7 g), and cross-linked polyvinylpyrrolidone (40 g) were mixed and subjected to extrusion and spheronization as per Example 1. Microcrystalline cellulose (10 g) was used as anticaking agent during the spheronization. The microgranules obtained after drying in a fluid bed had the following by wt. per cent composition:

| | |
|---|---|
| Ursodeoxycholic acid | 79.5 |
| Microcrystalline cellulose | 15.8 |
| Hydroxypropyl cellulose | 0.7 |
| Cross-linked polyvinylpyrrolidone | 4.0 |

### Example 5

Ursodeoxycholic acid (806 g), microcrystalline cellulose (145 g), hydroxypropyl cellulose (6 g), and cross-linked polyvinylpyrrolidone (110 g) were mixed and subjected to extrusion and spheronization as per Example 1. Microcrystalline cellulose (10 g) was used as anticaking agent during spheronization. The microgranules obtained after drying in a fluid bed had the following by wt. per cent composition:

| | |
|---|---|
| Ursodeoxycholic acid | 74.8 |
| Microcrystalline cellulose | 14.4 |
| Hydroxypropyl cellulose | 0.6 |
| Cross-linked polyvinylpyrrolidone | 10.2 |

### Example 6

Ursodeoxycholic acid (605 g), microcrystalline cellulose (71 g) and hydroxypropyl cellulose (5 g) were mixed and subjected to extrusion and spheronization as per Example 1. The microgranules obtained after drying in a fluid bed had the following by wt. per cent composition:

| | |
|---|---|
| Ursodeoxycholic acid | 87.3 |
| Microcrystalline cellulose | 12.0 |
| Hydroxypropyl cellulose | 0.7 |

### Dissolution rate

The method followed was as described in F.U. IX, vol I, p. 416, at a stirring rate of 100 rpm, with phosphate buffer (900 ml) at pH 7.5, at 37°C. The solution was sampled at various times; the samples were filtered through 0.45 µm membrane and restored to their original volume by addition of fresh buffer.

Samples analysis was carried out by HPLC (C18-RP column, mobile phase: water/acetonitrile, flow rate: 1 ml/min, detector: refractometer).

The data provide evidence that the preparation procedure according to the present invention brings about a high flexibility of ursodeoxycholic acid dissolution kinetics. It follows that fast-releasing formulations (4 hrs max. for an almost total active ingredient release) as well as formulations releasing the active ingredient over 24 hrs can be obtained.

## Claims

1. Controlled release pharmaceutical compositions for ursodeoxycholic acid oral administration, characterized in that they are in the form of microgranules prepared by extrusion-spheronization said microgranules containing ursodeoxycholic acid, a plasticizer consisting of microcrystalline cellulose, a binder selected from the group consisting of hydroxypropyl cellulose, hydroxypropyl methylcellulose, or linear polyvinylpyrrolidone, and optionally a disintegrating agent selected from the group consisting of cross-linked polyvinylpyrrolidone, cross-linked carboxymethylcellulose or carboxymethylamylum.

2. The pharmaceutical compositions according to claim 1 wherein the ursodeoxycholic acid content ranges between 50 and 90%, the plasticizer content between 5 and 50%, the binder content between 0.5 and 4%, the disintegrating agent content between 1 and 10% by wt.

3. The pharmaceutical composition according to any one of claims 1 and 2 wherein the ursodeoxucholic content is comprised between 70 and 90%, the plasticizer content between 10 and 30%, the binder content between 2 and 4%, the disintegrating agent between 1 and 5%.

4. A process for the preparation of the pharmaceutical compositions according to any one of claims 1-3, based on the extrusion of a mixture consisting of the drug, plasticizer, binder, optionally disintegrating agent and granulation liquid, followed by spheronization of the extrudate and drying of the resulting microgranules.

## Patentansprüche

1. Pharmazeutische Zusammensetzungen mit kontrollierter Freisetzung für die orale Verabreichung von Ursodeoxycholsäure, dadurch gekennzeichnet, daß sie in Form von Mikrokörnchen (Mikrogranulaten) vorliegen, die hergestellt worden sind durch Extrusions-Spheronisation der genannten Mikrokügelchen, die Ursodeoxycholsäure, einen Weichmacher, bestehend aus mikrokristalliner Cellulose, ein Bindemittel, ausgewählt aus der Gruppe, die besteht aus Hydroxypropylcellulose, Hydroxypropylmethylcellulose und linearem Polyvinylpyrrolidon, sowie gegebenenfalls ein Desintegrationsmittel, ausgewählt aus der Gruppe, die besteht aus vernetztem Polyvinylpyrrolidon, vernetzter Carboxymethylcellulose und Carboxymethylstärke, enthalten.

2. Pharmazeutische Zusammensetzungen nach Anspruch 1, worin der Gehalt an Ursodeoxycholsäure in dem Bereich zwischen 50 und 90 Gew.-%, der Weichmacher-Gehalt zwischen 5 und 50 Gew.-%, der Bindemittel-Gehalt zwischen 0,5 und 4 Gew.-% und der Gehalt an Desintegrationsmittel zwischen 1 und 10 Gew.-% liegen.

3. Pharmazeutische Zusammensetzung nach einem der Ansprüche 1 und 2, worin der Gehalt an Ursodeoxycholsäure zwischen 70 und 90 %, der Weichmacher-Gehalt zwischen 10 und 30 %, der Bindemittel-Gehalt zwischen 2 und 4 % und der Gehalt an Desintegrationsmittel zwischen 1 und 5 % liegen.

4. Verfahren zur Herstellung der pharmazeutischen Zusammensetzungen nach einem der Ansprüche 1 bis 3, das basiert auf der Extrusion einer Mischung, die besteht aus dem Arzneimittel (Wirkstoff), dem Weichmacher, dem Bindemittel, gegebenenfalls dem Desintegrationsmittel und der Granulationsflüssigkeit, woran sich die Spheronisation des Extrudats und das Trocknen der resultierenden Mikrokörnchen (Mikrogranulate) anschließen.

## Revendications

1. Compositions pharmaceutiques à libération contrôlée pour l'administration orale de l'acide ursodésoxycholique, caractérisées en ce qu'elles sont sous forme de microgranules préparées par extrusionsphéronisation, lesdites microgranules contenant de l'acide ursodésoxycholique, un plastifiant consistant en cellulose microcristalline, un liant choisi au sein du groupe comprenant l'hydroxypropylcellulose, l'hydroxypropylméthylcellulose ou la polyvinylpyrrolidone linéaire et éventuellement un agent de délitescence choisi au sein du groupe comprenant la polyvinylpyrrolidone réticulée, la carboxyméthylcellulose réticulée ou le carboxyméthylamylum.

2. Compositions pharmaceutiques selon la revendication 1, dans lesquelles la teneur en acide ursodésoxycholique est comprises entre 50 et 90 %, la teneur en plastifiant est comprise entre 5 et 50 %, la teneur en liant est comprise entre 0,5 et 4 %, et la teneur en agent de délitescence est comprise entre 1 et 10 % en poids.

3. Composition pharmaceutique selon l'une quelconque des revendications 1 à 2, dans laquelle la teneur en l'acide ursodésoxycliolique est comprise entre 70 et 90 %, la teneur en plastifiant entre 10 et 30 %, la teneur en liant entre 2 et 4 %, la teneur en agent de délitescence entre 1 et 5 %.

4. Procédé de préparation des compositions pharmaceutiques selon l'une quelconque des revendications 1 à 3, basé sur l'extrusion d'un mélange comprenant le médicament, le plastifiant, le liant, éventuellement l'agent de délitescence et le liquide de granulation, suivie de la sphéronisation de l'extrudat et du séchage des microgranules ainsi obtenues.
